# EUROPEAN PATENT APPLICATION

(11) **EP 2 932 909 A1**
(43) Date of publication of application: **21.10.2015**
(21) Application number: 14199674.4
(22) Date of filing: 22.12.2014
(51) Int. Cl.: A61B 17/00

(54) **Specimen retrieval pouch having a lubricious coating**

(30) Priority: 16.04.2014 US 201461980214 P; 07.10.2014 US 201414507871
(71) Applicant: Covidien LP, Mansfield, MA 02048 (US)
(72) Inventor: Horton, Kenneth W., South Glastonbury, Connecticut 06073 (US)
(74) Representative: Soames, Candida Jane

(57) **Abstract**

A lubricious material is provided on at least a portion of the exterior surface of a specimen retrieval pouch.

## Description

### CROSS-REFERENCE TO RELATED APPLICATIONS

This application claims the benefit of and priority to U.S. Provisional Patent Application No. 61/980,214, filed April 16, 2014, the entire disclosure of which is incorporated by reference herein.

### BACKGROUND

### Technical Field

The present disclosure relates to a specimen retrieval pouch. More particularly, the present disclosure relates to a specimen retrieval pouch having a lubricious coating on at least a portion of the outer surface thereof.

### Background of Related Art

Minimally invasive procedures may be used for partial or total removal of body tissue or organs from the interior of the body, e.g. nephrectomy, cholecystectomy, lobectomy and other procedures including thoracic, laparoscopic and endoscopic procedures. During such procedures, it is common that a tissue specimen (e.g., cyst, cancerous tissue, or other affected tissue or organ) be removed from the patient's body via an access opening in the skin, or through an access port, e.g., a cannula.

Devices for removing a tissue specimen from the patient's body typically include a pouch (or other suitable flexible container) releasably supported at a distal end of the device. Manipulating the pouch having a tissue specimen therein can sometimes prove difficult within the confined space of a body cavity. Similarly, removing the pouch through an access opening in the skin or access port that is smaller than the tissue specimen contained within the pouch can also prove difficult.

In minimally invasive video-assisted thoracic surgery (VATS), for example, it is sometimes necessary to remove a pouch and tissue specimen contained therein through an access opening (or port) that has been positioned between a patient's ribs to provide access to a thoracic cavity of the patient. However, the restricted space between the patient's ribs may make it may difficult for a surgeon to pull the pouch and tissue specimen contained therein through the access opening. Likewise, the restricted space between the patient's ribs may make it difficult for a surgeon to maintain the tissue specimen relatively intact (e.g., for pathology).

### SUMMARY

Specimen retrieval devices in accordance with the present disclosure include a pouch having a lubricious coating on at least a portion of the outer surface thereof. The lubricious coating on at least a portion of the outer surface of the pouch facilitates manipulation of the pouch within a surgical site and/or removal of the pouch from a body cavity of a patient.

An aspect of the present disclosure provides a specimen retrieval pouch which is couplable to an applicator of a specimen retrieval device for removing a tissue specimen from a body cavity of a patient. The specimen retrieval pouch includes a closed bottom portion and an open upper portion configured to receive the tissue specimen. A lubricious material is present on at least a portion of the outer surface of the specimen retrieval pouch.

The lubricious material present on at least a portion of the outer surface of the specimen retrieval pouch may be a hydrophilic material or a hydrophobic material. In embodiments, at least a portion of the outer surface of the specimen retrieval pouch is coated with the lubricious material. In embodiments, at least a portion of the pouch is formed from the lubricious material, thus providing a lubricious surface on at least a portion of the outer surface of the specimen retrieval pouch.

In embodiments, the exterior surface of the pouch may be evenly coated with the lubricious material, which may include antithrombogenic properties. In embodiments, the upper portion of the pouch may be coated with a more lubricious material than the closed bottom portion. In addition to lubricious material being present on at least a portion of the outer surface of the specimen retrieval pouch, in embodiments, at least a portion of the interior surface of the pouch is coated with a lubricious material. In embodiments, the pouch may be formed from the hydrophobic material and at least a portion of the outer surface of the pouch may be coated with the hydrophilic material. In embodiments, the lubricious material may include synthetic and/or natural materials, e.g., hydrogels. For example, the lubricous material may include one or more of the following: glycerine, silicone oil, olive oil, fluoropolymers, hyaluronan, cyanoacrylates, epoxies, polyalkylene oxides, urethanes, siloxanes, parylenes, polyacrylamides, poly-N-vinyl pyrrolidone (PVP), polyvinyl alcohol, polyvinyl methyl ether, polyethylene oxide (PEO), polyethylene glycol (PEG), polyacrylic acid and polyacrylic acid salts, chemical derivatives of cellulose, such as carboxymethylcellulose. It is also contemplated that other lubricious coatings and materials known to those of skill in the art may be incorporated.

In embodiments, the specimen retrieval pouch is formed from a porous polymeric material. In such embodiments, the lubricious material may impregnate the porous material, rendering the pouch impermeable.

In embodiments, the exterior surface of the specimen retrieval pouch is modified by chemical solution etching, plasma gas etching, or plasma polymerization deposition.

The lubricious material may be non-thrombogenic. Moreover, the lubricious material may prevent adhesion of platelets, proteins, microorganisms, adsorbed platelets, proteins, bacteria, and/or prevent acute thrombus formation.

### BRIEF DESCRIPTION OF THE DRAWINGS

Embodiments of the presently disclosed specimen retrieval device are described hereinbelow with reference to the drawings wherein:
FIG. 1 is a perspective view of a specimen retrieval device and a specimen retrieval pouch in accordance with an embodiment of the present disclosure;
FIG. 2 is a perspective view of the specimen retrieval device shown in FIG. 1 with a portion cut away to show the specimen retrieval pouch in a retracted configuration;
FIGS. 3A and 3B are perspective views of specimen retrieval devices including specimen retrieval pouches according to alternate embodiments of the present disclosure;
FIGS. 4A-4D are schematic views of specimen retrieval pouches according to alternate embodiments of the present disclosure;
FIG. 5A is a perspective view of a specimen retrieval device inserted into a thoracic cavity of a patient with the specimen retrieval pouch having tissue positioned therein and in a cinched configuration; and
FIG. 5B is a perspective view of the cinched pouch shown in FIG. 4A being pulled through an incision in the patient.

### DETAILED DESCRIPTION OF THE EMBODIMENTS

Embodiments of the present disclosure will now be described in detail with reference to the drawings, in which like reference numerals designate identical or corresponding elements in each of the several views. As used herein, the term "distal," as is conventional, will refer to that portion of the instrument, apparatus, device or component thereof which is farther from the user while, the term "proximal," will refer to that portion of the instrument, apparatus, device or component thereof which is closer to the user. In the following description, well-known functions or constructions are not described in detail to avoid obscuring the present disclosure in unnecessary detail.

As used herein with reference to the present disclosure, the terms laparoscopic and endoscopic are interchangeable and refer to instruments having a relatively narrow operating portion for insertion into a cannula or a small incision in the skin. Throughout the present disclosure, the term "minimally invasive" should be understood to encompass both endoscopic and laparoscopic procedures. It is believed that the devices of the present disclosure may find use in any procedure where access to the interior of the body is limited to a relatively small incision, with or without the use of a cannula, as in minimally invasive procedures.

A pouch in accordance with the instant disclosure resists tearing or rupture while providing sufficient rigidity and/or maneuverability. Additionally, the pouch reduces collateral damage to tissue during manipulation of the pouch within and/or removal of the pouch from a body cavity of a patient. Further, the pouch is capable of removing large tissue specimens through small access incisions or ports while minimizing the risk of contaminating the body cavity with the cells from the tissue sample being removed (e.g., seeding of cancer cells when a tumor is being removed).

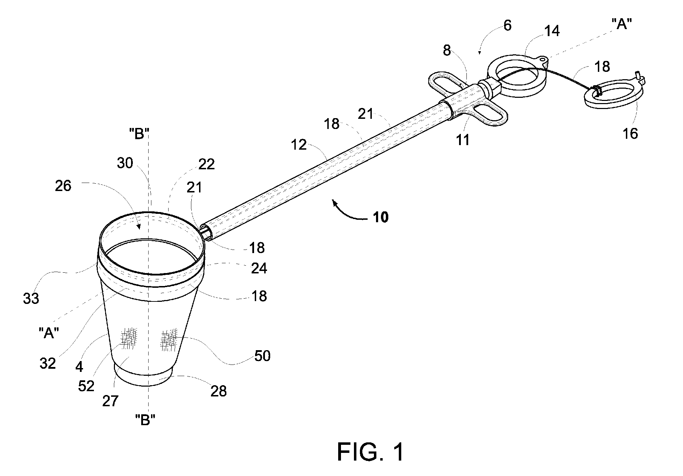

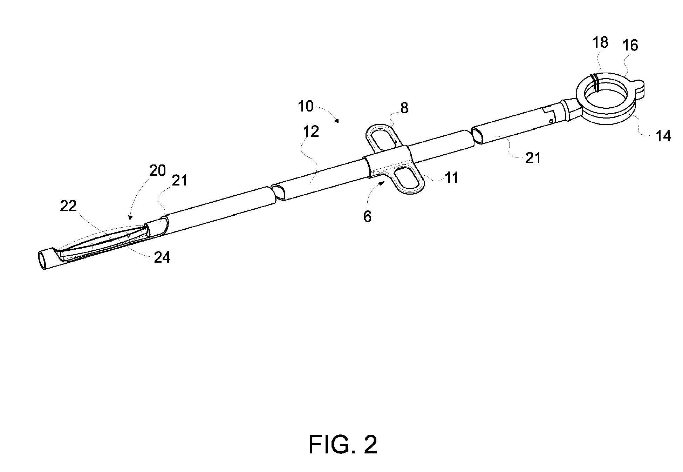

With reference to FIGS. 1 and 2, a specimen retrieval device 10 including a specimen retrieval pouch 4 having a lubricious material on at least a portion of the outer surface thereof according to an embodiment of the present disclosure is illustrated. Briefly, specimen retrieval device 10 includes a handle assembly 6 that includes handle portions 8 and 11 that are fixedly joined together. An elongated tube or shaft 12 extends from handle assembly 6 and is dimensioned for insertion through a trocar cannula (not explicitly shown) or access opening in a patient for endoscopic or laparoscopic procedures. A longitudinal axis "A-A" is defined through shaft 12 and is oriented in a substantially perpendicular or orthogonal direction with respect to a longitudinal axis "B-B" that is defined through pouch 4 when pouch 4 is in the deployed state.

A drive rod 21 is positioned within shaft 12 and is movable relative to shaft 12 to move pouch 4 between deployed and retracted positions (FIGS. 1-2). Drive rod 21 includes a proximal end that operably couples to an actuator that is in the form of a finger loop 14. Finger loop 14 is configured for engagement by a user's fingers to translate or move drive rod 21 within shaft 12 between the deployed and retracted configurations. A pull ring 16 releasably couples, via one or more suitable coupling methods, e.g., a press or friction fit, to finger loop 14 and is coupled (e.g., tied, adhesive, etc.) to a proximal end of a drawstring 18 to facilitate pulling the drawstring 18. A distal end of drawstring 18 is operably coupled to open upper portion 26 of pouch 4.

Drive rod 21 includes a distal end which supports a resilient, deformable spring 20 including two generally flexible or resilient strips 22 and 24 (FIG. 2). Resilient strips 22, 24 of spring 20 extend from the distal end of drive rod 21 and operably couple to pouch 4. Resilient strips 22, 24 move from a stressed or non-expanded state to an unstressed or freely expanded state when pouch 4 is moved from the retracted position (FIG. 2) to the deployed position (FIG. 1). In the stressed or non-expanded state, pouch 4 is wound or wrapped around resilient strips 22 and 24. Wrapping pouch 4 around resilient strips 22 and 24 facilitates deploying pouch 4 from the relatively small area within shaft 12. In an unstressed or freely expanded condition, resilient strips 22 and 24 collectively form a generally circular or "hoop-like" configuration for supporting a periphery of open upper portion 26 of pouch 4 (see FIG. 1).

For a more detailed description of specimen retrieval device 10 and operative components associated therewith, reference is made to U.S. Patent No. 5,647,372, the disclosure of which is incorporated herein in its entirety by this reference.

With reference again to FIG. 1, pouch 4 includes a generally tubular or elongated configuration that is defined by open upper portion 26 and closed lower portion 28 and has a lubricious material on at least a portion of the outer surface thereof. Open upper portion 26 includes a proximal (or upper) circumferential tubular portion or sleeve 30 and a distal (or lower) circumferential tubular portion or sleeve 32, which are spaced apart from each other. Proximal and distal sleeves 30 and 32, respectively, are configured to receive spring 20 and drawstring 18 as seen in FIG. 1. A linear portion 33 weakened by perforation, scoring, or the like, extends circumferentially around open upper portion 26 and is operably disposed between the proximal and lower sleeves 30 and 32. Linear portion 33 is adapted to tear when drawstring 18 is pulled proximally with sufficient force to close open upper portion 26 of pouch 4 distal to linear portion 33, thereby providing fast detachment of pouch 4 from spring 20 simultaneously with closure of open upper portion 26 of pouch 4. Thus, open upper portion 26 of pouch 4 is openable by spring 20 upon deployment and closable by drawstring 18 upon separation from spring 20. Those skilled in the art reading this disclosure will appreciate that alternative methods also can be utilized to detach pouch 4 from spring 20, such as by pulling with a grasper or by cutting with a scissors.

Pouch 4 is configured for the purpose of tissue entrapment and/or removal. In embodiments, pouch 4 may have a diameter that ranges from about 1.5 inches to about 3.0 inches and a changeable or variable depth that ranges from about 2 inches to about 8 inches.

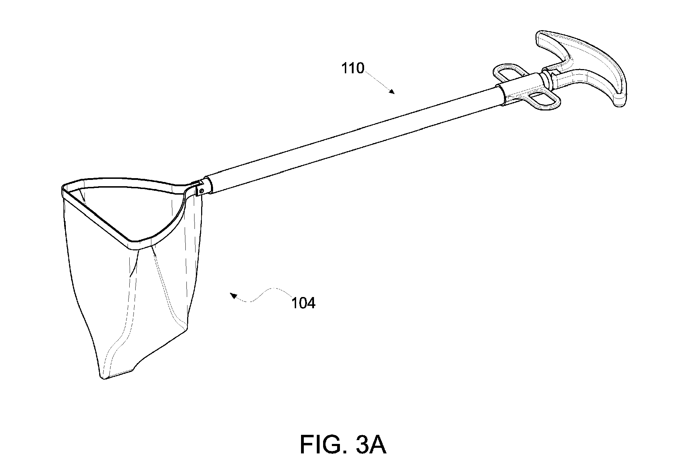

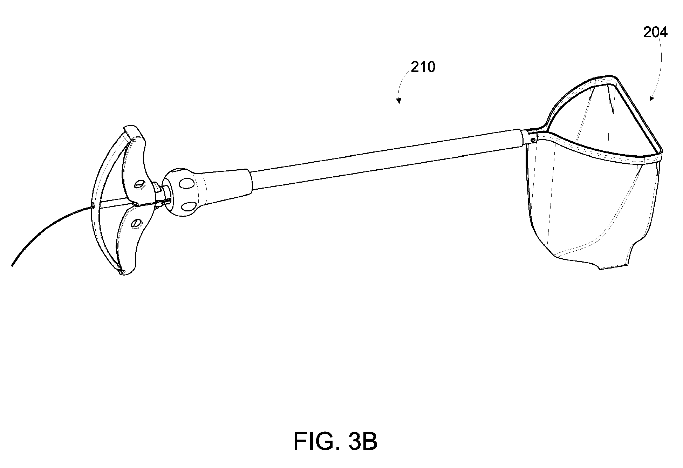

FIGS. 3A and 3B illustrate specimen retrieval devices 110, 210, respectively, that may be configured for use with pouches 104, 204. Other than the shape of the pouches 104, 204 and the manner in which the pouches 104, 204 may be coupled to and/or manipulated by the specimen retrieval devices 110, 210, pouches 104 and 204 are configured to function in a manner substantially similar to that of pouch 4. Other shapes for pouches 4, 104, 204 are also contemplated including square, rectangular, oval, etc.

Pouch 4 may be made from any suitable flexible, substantially impervious biocompatible material (e.g., impervious to penetration by cells, such as cancer cells). In embodiments, for example, pouch 4 may be formed from a flexible film or sheet formed from a substantially transparent polymeric material. In embodiments, the polymeric material may be a polyurethane sheet having a thickness ranging from about 0.001 inches to about 0.005 inches. In other embodiments, pouch 4 is made from a material that includes interstices, and application of a lubricious material penetrates and seals the interstices, rendering pouch 4 impervious to penetration by cells, etc.

In accordance with the present disclosure, a lubricious material is present on at least a portion of the outer surface of pouch 4. In embodiments, at least a portion of the outer surface of pouch 4 is coated with a lubricious material. In other embodiments, pouch 4 is made at least in part from a lubricious material.

Any suitable lubricious material may be present on at least a portion of outer surface of the pouch. Such materials include, for example, cyanoacrylates; epoxies; hydrophilic polymers including hydrogels; polyalkylene oxides including polyethylene oxide, polypropylene oxide, polyethylene oxide/polypropylene oxide copolymers, polyethylene glycol, polypropylene glycol; urethanes such as polyurethane; siloxanes, including cyclosiloxanes, polyalkylsiloxanes, and polydialkylsiloxanes such as polydimethylsiloxanes, polydiethylsiloxanes, polydipropylsiloxanes, polydibutylsiloxanes; parylenes including parylene N, parylene C, parylene D, and parylene HT; combinations thereof, and the like. One suitable lubricious material is disclosed in United States Patent 7,141,246.

Any means within the purview of those skilled in the art may be utilized to apply the lubricious material to the pouch. In some embodiments, suitable methods which may be utilized to apply the lubricious material include gravure coating, spray coating, melt blowing, thermal/heat transfer, vapor deposition, deposition by nozzle, flexographic printing techniques, such as offset flexographic printing, screen printing, laserjet or inkjet, combinations thereof, and the like. Relevant parameters related to application include, but are not limited to, nozzle geometry of a spray device, time necessary for application, pressure, temperature, humidity, curing time and temperature, wavelength where curing includes the application of light, the state of the substrate, including its natural or elongated state, the coating thickness, as well as geometry, size and spacing of any pattern employed, and the like.

In embodiments, the materials utilized to form the lubricious coatings of the present disclosure may be applied in powder form. In other embodiments, the materials utilized to form the lubricious coatings of the present disclosure may be applied in liquid form. Where the materials are applied as liquids, it may be desirable to form a solution by placing the materials utilized to form the lubricious coatings of the present disclosure in a suitable solvent. Any solvent within the purview of those skilled in the art may be utilized; however, as would be apparent to one skilled in the art, the solvent should be compatible with both the lubricious coating materials and the material from which the pouch is made and should not degrade either during application of the lubricious material. Examples of suitable solvents include tetrahydrofuran, chlorinated hydrocarbons such as methylene chloride, alcohols such as methanol, ethanol, and propanol, chloroform, 1,2-dichloro-ethane, aliphatic hydrocarbons such as hexane, heptene, and ethyl acetate, combinations thereof, and the like.

Where the lubricious material is applied as part of a solution, the coating solution may contain from about 30 to about 70 weight percent solvent, in embodiments from about 45 to about 55 weight percent solvent. If desired, the coating solution can optionally contain additional components such as dyes, antibiotics, antiseptics, growth factors, anti-inflammatory agents, combinations thereof, and the like.

In embodiments, anti-microbial additives may be provided within the lubricious material and released during use of the pouch. Suitable anti-microbial additives include, but are not limited to, the biguanides (such as chlorhexidine and its salts, including chlorhexidine acetate, chlorhexidine gluconate, chlorhexidine hydrochloride, and chlorhexidine sulfate), silver and its salts (such as silver acetate, silver benzoate, silver carbonate, silver iodate, silver iodide, silver lactate, silver laurate, silver nitrate, silver oxide, silver palmitate, silver protein, and silver sulfadiazine), polymyxin, tetracycline, aminoglycosides (such as tobramycin and gentamicin), rifampician, bacitracin, neomycin, chloramphenical, miconazole, tolnaftate, quinolones (such as oxolinic acid, norfloxacin, nalidix acid, pefloxacin, enoxacin and ciprofloxacin), penicillins (such as ampicillin, amoxicillin and piracil), cephalosporins, vancomycin, extracts of plants and herbs known to possess anti-microbial activity (e.g., phytochemicals, such as grapefruit seed extract, Tea Tree Oil and Myrtle Oil) and combinations of any of the above anti-microbials.

Once applied, in some embodiments the materials utilized to provide a lubricious material on at least a portion of the outer surface of the pouch in accordance with the present disclosure may be further treated to further enhance formation of the coating. Methods for treating the materials utilized to form lubricious coatings of the present disclosure are within the purview of those skilled in the art. For example, in some embodiments it may be desirable to cure the coating materials to enhance their lubricity. Such methods for curing may include heating to suitable temperatures, which will depend upon the materials utilized to form the coatings herein. This may be desirable where the coating materials are applied in powder form, or where the coating materials are applied in solution and it is desired to remove the solvent utilized to form the solution. For example, once applied, the coating material and the now-coated pouch may be heated to a temperature of from about 80° F to about 350° F, in embodiments from about 120° F to about 250° F. A vacuum may be applied in embodiments to accelerate curing. In other embodiments, solvents utilized to apply the coatings may simply evaporate upon application, leaving the lubricious material upon the outer surface of the pouch.

In other embodiments, curing may occur by the application of a light source including ultraviolet light or light from a coherent light source such as a laser source. Suitable UV light sources may be at a wavelength of from about 80 nm to about 400 nm, in embodiments from about 290 nm to about 380 nm. Suitable coherent light sources such as a laser may be applied at a wavelength of from about 80 nm to about 1540 nm, in embodiments from about 290 nm to about 400 nm.

The lubricious material may be applied over the entire outer surface of the pouch or, alternatively, on only a portion of outer surface of pouch. When applied to only a portion of outer surface of the pouch, the lubricious material may be applied in any suitable pattern, including dots, stripes, spirals, combinations thereof, and the like. The spacing, pattern, and geometry utilized in applying the lubricious material may vary to accommodate the geometry of the pouch.

In embodiments where the lubricious material is present on only a portion of the outer surface of the pouch, a mask material may be applied to the pouch, wherein the mask has a configuration of the pattern of lubricious material to be applied to the outer surface of the pouch. The masking material is first applied to the outer surface of the pouch, having openings therein corresponding to the pattern to be applied to the outer surface of the pouch. The coating materials may then be applied utilizing any method within the purview of those skilled in the art, including those noted above, as well as dipping, the pouch into the coating material. The coating materials may then be allowed to cure, if necessary, after which time the masking material may be removed, leaving the pattern of lubricious material on the outer surface of the pouch.

In embodiments, lubricious material may be present on an outer surface of the pouch in a pattern that may have a coating-free surface area of more than about 25% of the surface, in embodiments a coating-free surface area of from about 50% to about 75% of the surface, in other embodiments a coating-free surface area of from about 75% to about 90%.

Thus, the presence of the lubricious material on at least a portion of the surface of the pouch may be utilized to transform the surface of a pouch having a surface with a higher coefficient of friction into a pouch with a surface having a lower coefficient of friction. While the lubricious material itself may be rigid, pouches made of flexible substrates possessing a coating of the lubricious material may remain flexible without substantial fracturing of the coating or delamination of the coating from the pouch.

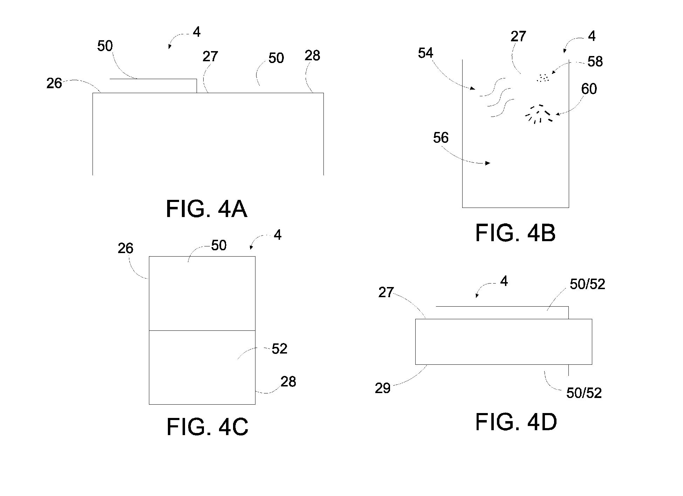

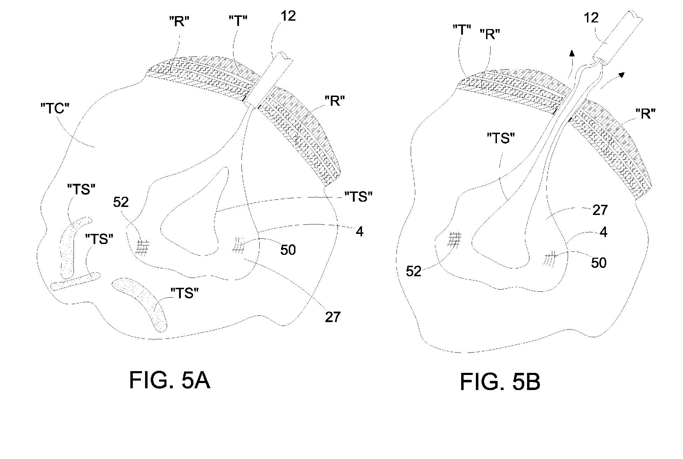

In embodiments, an external surface 27 of pouch 4 is coated with a biocompatible hydrophilic material 50 (represented by hatching in FIGS. 1, 5A and 5B) that becomes lubricious when wet. When wet, the lubricity of hydrophilic material 50 facilitates manipulation of pouch 4 within a body cavity and/or facilitates removal of pouch 4 through an access opening in the skin (FIG. 4B), or through a cannula, which, in turn, may prevent or reduce trauma to tissue and/or tissue abrasion. The thickness of the coating of lubricious material may be from about 1 µm to about 20 µm for a pouch having a wall thickness of from about 0.001 inches to about 0.005 inches.

Hydrophilic material 50 may be water-soluble or non-water soluble. Additionally, hydrophilic material 50 may be synthetic or natural. In embodiments, hydrophilic material 50 is a water-soluble synthetic polymer. Such water-soluble polymers include polyacrylamides, polyvinyl pyrrolidone (PVP), polyvinyl alcohol, polyvinyl methyl ether, polyethylene oxide, polyethylene glycol, polyacrylic acid and their salts, and various chemical derivatives of cellulose, such as carboxymethylcellulose.

In embodiments, hydrophilic material 50 may be synthetic absorbable polymers that are water-soluble; that is, the polymers migrate from the site by a combination of absorption and water dissolution. Members of the polyoxaester coating family, based on polyglycol diacid, and polyethylene glycol diols, provide such features. In one particular embodiment, the polyoxaester can be prepared by polycondensation using polyethylene glycol diol with a molecular weight of 200 Daltons, and a small amount of ethylene glycol and polyglycol diacid having a molecular weight of about 600 Daltons. The resulting polyester is both absorbable and water-soluble. The molecular weight of the polyester can be controlled by conventional means. Higher molecular weight resin will remain for a longer period of time on the pouch 4. Alternately, the polyoxaester can be further modified by polymerization with lactone monomers using ring-opening techniques. Useful lactone monomers include glycolide, lactide, p-dioxanone, trimethylene carbonate and ε-caprolactone. Of particular utility is copolymerization with glycolide. Copolymerizing with lactone will alter water solubility and dissolution rates, as well as absorption rates.

In embodiments, the lubricious material may be a hydrophobic material 52 (FIG. 1). Hydrophobic material 52 may include, but is not limited to, silicones and salts of fatty acids, such as calcium stearate. Alternatively, in embodiments, the lubricous material may be a combination of hydrophilic material 50 and hydrophobic material 52, e.g., amphiphilic material.

Other natural and synthetic materials not described herein could advantageously be employed according to the present disclosure. The above lists of natural and synthetic materials are not exhaustive and are intended for illustrative purposes only. There are an endless variety of natural and synthetic materials which may be utilized for the lubricious material on pouch 4.

In embodiments, exterior surface 27 of pouch 4 may be evenly coated with the hydrophilic material 50. Alternatively, it may prove advantageous to coat exterior surface 27 with more hydrophilic material 50 at open upper portion 26 (e.g., adjacent perforation 33) than at closed bottom portion 28 of pouch 4 (shown schematically in FIG. 4A). As can be appreciated, other coating configurations may also be utilized to coat exterior surface 27 of pouch 4; such as alternating patterns 54, stripes 56, dots 58, random patterns 60, etc. (see FIG. 4B for example).

In embodiments, it may prove advantageous to coat exterior surface 27 with a hydrophilic material 50 (or hydrophobic material 50) at open upper portion 26 (e.g., adjacent perforation 33) that is more lubricious than a hydrophilic material 50 (or hydrophobic material 50) that coats closed bottom portion 28 of pouch 4. Alternatively, open upper portion 26 of pouch 4 may be coated with a lubricious material and closed bottom portion 28 of pouch 4 may be uncoated.

In embodiments, it may prove advantageous for both hydrophilic and hydrophobic material 50 to be present on exterior surface 27 of pouch 4. In this particular embodiment, a top half of exterior portion 27 of pouch 4 may be coated with hydrophilic material 50 and a bottom half of exterior portion 27 of pouch 4 may be coated with hydrophobic material 50 (or vice versa) (see FIG. 4C).

In embodiments, the thickness, wettability, lubricity, flexibility, abrasion resistance, and/or storage stability of the hydrophilic material 50 may be varied to meet the specific requirements of a manufacturer and/or specific medical procedure.

In embodiments, the pouch can be made from a porous polymeric material, such as ePTFE, and the outer surface of the porous polymeric material forming the pouch is impregnated with a lubricious material of the type described above in connection with previous embodiments.

The outer surface of the porous polymeric material forming the pouch may be impregnated by applying a solution of the lubricious materialIn an exemplary process, a pouch made of a porous polymeric material (e.g., ePTFE) is cleaned, for example by argon plasma cleaning (e.g., by subjecting the pouch to the plasma at about 200 watts for about 5 minutes). A solution of the lubricious material is applied to the outer surface of the cleaned porous polymeric pouch, in one or more applications. In embodiments, enough solution to flood and saturate the surface of the cleaned porous polymeric pouch is applied. The pouch, wet with the solution, is then dried to evaporate the solution solvent, leaving the lubricious material impregnated in the porous polymeric pouch and on at least a portion of the exterior surface of the pouch.

In an alternative embodiment, the outer surface of the porous polymeric pouch is modified by depositing a functionality on the porous polymeric material, for example by treating the outer surface with a treatment selected from the group consisting of chemical solution etching, plasma gas etching, and plasma polymerization deposition.

As those skilled in the art reading this disclosure will appreciate, chemical solution etching process typically involves dipping into, or otherwise exposing the outer surface of the porous polymeric pouch to, a chemical etch solution (such as, for example, a sodium-naphthalate chemical etch solution). After removal from the etching solution, the porous polymeric pouch is typically dipped or otherwise rinsed in a solution such as isopropyl alcohol to quench/deactivate any remaining etching solution thereon. The quenching solution is then rinsed away using warm water and the resulting etched pouch is dried. A lubricious material may then be applied to the resulting etched pouch as set forth above.

In an alternative embodiment, the outer surface of the pouch is prepared using a gas plasma etch/treatment, such as an ammonia gas plasma treatment. In the ammonia gas plasma treatment, the surface is treated with ammonia anions by reaction in an ammonia gas filled plasma chamber, to deposit an amine functionality on the surface of the porous polymeric pouch. Alternative gases may be used in the gas plasma etching in addition to or instead of the ammonia gas. In one embodiment, the porous polymeric pouch is gas plasma etched by placing it in a plasma chamber and exposing it to the ammonia plasma for about 1 to about 5 minutes with the ammonia plasma generated at a power of about 450 watts. A lubricious material may then be applied to the resulting plasma treated pouch as set forth above.

In an alternative embodiment, the outer surface of the pouch is prepared by depositing a plasma polymerized functionality. In embodiments, the modified outer surface is a plasma polymerized carboxylate film including an acrylate or acrylate-like polymer layer deposited onto the porous polymeric pouch by exposing the porous polymeric pouch to a plasma, such as, for example, an acrylic acid plasma. A lubricious material may then be applied to the resulting modified outer surface as set forth above.

It should, of course, be understood that the foregoing surface modification techniques may also be applied to non-porous pouch prior to application of a lubricious material.

The specimen retrieval device including pouch 4 may utilized in various surgical procedures. In embodiments, for example, specimen retrieval device 10 including pouch 4 may be utilized in conjunction with video assisted thorascopic surgery (VATS).

In such embodiments, an incision may, initially, be made in tissue "T" of a patient to access the thoracic cavity "TC" (FIG. 5A). Shaft 12 of specimen retrieval device 10 may be inserted through the incision in the patient and between ribs "R" of the patient to access and position the pouch within the thoracic cavity "TC."

Graspers (not shown) may be utilized to position one or more tissue specimens "TS," which were previously excised from a lung of the patient, within pouch 4. Thereafter, drawstring 18 may be pulled proximally to cinch and secure tissue specimen "TS" within pouch 4. Once cinched, pouch 4, including tissue specimen "TS" therein, may be pulled between ribs "R" and through the incision in a patient (FIG. 5B).

Lubricious hydrophilic material 50 (or hydrophobic material 52) provided on exterior surface 27 of pouch 4 allows for easy maneuverability of the pouch 4 within the thoracic cavity "TC" and removal of pouch 4 from between the ribs "R" and through the incision in the patient. As can be appreciated, this, in turn, may result in less patient trauma and/or less postoperative pain to a patient.

From the foregoing and with reference to the various figure drawings, those skilled in the art will appreciate that certain modifications can also be made to the pouch 4 of the present disclosure without departing from the scope of the same. For example, it may prove advantageous to coat an interior surface 29 of pouch 4 with hydrophilic coating 50 (or hydrophobic material 52) in addition to coating exterior surface 27 of pouch 4 with hydrophilic coating 50 (or hydrophobic material 52) (see FIG. 4D for example).

In embodiments, it may prove advantageous for form pouch 4 from a hydrophobic material 52 (e.g., silicone) and coat exterior surface 27 of pouch 4 with one of the aforementioned hydrophilic materials 50 (e.g., polyacrylamides).

In embodiments, pouch 4 may be dimensioned and fabricated of a suitable material to allow treatment, e.g. morcellation or division, of the organ tissue, for example to reduce its bulk to facilitate withdrawal from the body cavity.

In embodiments, hydrophilic material 50 may be abrasion resistant or non-thrombogenic. In embodiments, hydrophilic material 50 may be configured to prevent adhesion of platelets, proteins, microorganisms, adsorbed platelets, proteins, bacteria, and/ or acute thrombus formation.

While several embodiments of the disclosure have been shown in the drawings, it is not intended that the disclosure be limited thereto, as it is intended that the disclosure be as broad in scope as the art will allow and that the specification be read likewise. Therefore, the above description should not be construed as limiting, but merely as exemplifications of particular embodiments. Those skilled in the art will envision other modifications within the scope and spirit of the claims appended hereto.

The invention may be described by reference to the following numbered paragraphs:-
1. A specimen retrieval pouch, comprising:
   a closed bottom portion and an open upper portion configured to receive a tissue specimen, at least a portion of an exterior surface of the specimen retrieval pouch including a lubricious material.
2. A specimen retrieval pouch according to paragraph 1, wherein at least a portion of the exterior surface of the specimen retrieval pouch is coated with the lubricious material.
3. A specimen retrieval pouch according to paragraph 1, wherein at least a portion of the specimen retrieval pouch is formed from the lubricious material to provide lubricious material on at least a portion of an exterior surface of the specimen retrieval pouch.
4. A specimen retrieval pouch according to paragraph 1, wherein the lubricious material is a hydrophilic material.
5. A specimen retrieval pouch according to paragraph 1, wherein the lubricious material is selected from the group consisting of polyacrylamides, polyvinyl pyrrolidone (PVP), polyvinyl alcohol, polyvinyl methyl ether, polyethylene oxide, polyethylene glycol, polyacrylic acid, polyacrylic acid salts, and chemical derivatives of cellulose.
6. A specimen retrieval pouch according to paragraph 1, wherein the lubricious material is uniformly present on the exterior surface of the specimen retrieval pouch.
7. A specimen retrieval pouch according to paragraph 1, wherein the open upper portion of the specimen retrieval pouch is coated with a lubricious material and the closed bottom portion of the specimen retrieval pouch is uncoated.
8. A specimen retrieval pouch according to paragraph 1, wherein at least a portion of an interior surface of the specimen retrieval pouch is coated with a lubricious material.
9. A specimen retrieval pouch according to paragraph 1, wherein the pouch is formed from a hydrophobic material.
10. A specimen retrieval pouch according to paragraph 4, wherein the pouch is formed from a hydrophobic material.
11. A specimen retrieval pouch according to paragraph 1, wherein the pouch is formed from a porous polymeric material.
12. A specimen retrieval pouch according to paragraph 10, wherein the exterior surface of the specimen retrieval pouch is modified by chemical solution etching, plasma gas etching, or plasma polymerization deposition.
13. A specimen retrieval pouch according to paragraph 1, wherein the lubricious material is selected from the group consisting of cyanoacrylates, epoxies, polyalkylene oxides, urethanes, siloxanes, and parylenes.
14. A specimen retrieval pouch according to paragraph 1, wherein the lubricious material includes an anti-microbial additive.
15. A specimen retrieval device, comprising
   an applicator; and
   a specimen retrieval pouch operably couplable to the applicator, the specimen retrieval pouch including a closed bottom portion and an open upper portion configured to receive a tissue specimen, at least a portion of an exterior surface of the specimen retrieval pouch including a lubricious material.
16. A specimen retrieval device according to paragraph 15, wherein at least a portion of the exterior surface of the specimen retrieval pouch is coated with the lubricious material.
17. A specimen retrieval device according to paragraph 15, wherein at least a portion of the pouch is formed from the lubricious material to provide lubricious material on at least a portion of an exterior surface of the specimen retrieval pouch.
18. A specimen retrieval device according to paragraph 15, wherein the lubricious material is a hydrophilic material.
19. A method comprising:
   providing a specimen retrieval pouch including a closed bottom portion and an open upper portion configured to receive a tissue specimen; and
   applying a lubricious material to at least a portion of an exterior surface of the open upper portion of the specimen retrieval pouch.
20. A method according to paragraph 19, wherein the exterior surface of the specimen retrieval pouch is modified by chemical solution etching, plasma gas etching, or plasma polymerization deposition prior to applying the lubricious material.

## Claims

1. A specimen retrieval pouch, comprising:
a closed bottom portion and an open upper portion configured to receive a tissue specimen, at least a portion of an exterior surface of the specimen retrieval pouch including a lubricious material.

2. A specimen retrieval pouch according to claim 1, wherein at least a portion of the exterior surface of the specimen retrieval pouch is coated with the lubricious material; or wherein at least a portion of the specimen retrieval pouch is formed from the lubricious material to provide lubricious material on at least a portion of an exterior surface of the specimen retrieval pouch; preferably wherein the lubricious material is a hydrophilic material.

3. A specimen retrieval pouch according to claim 1 or claim 2, wherein the lubricious material is selected from the group consisting of polyacrylamides, polyvinyl pyrrolidone (PVP), polyvinyl alcohol, polyvinyl methyl ether, polyethylene oxide, polyethylene glycol, polyacrylic acid, polyacrylic acid salts, and chemical derivatives of cellulose.

4. A specimen retrieval pouch according to any preceding claim, wherein the lubricious material is uniformly present on the exterior surface of the specimen retrieval pouch.

5. A specimen retrieval pouch according to any of claims 1 to 4, wherein the open upper portion of the specimen retrieval pouch is coated with a lubricious material and the closed bottom portion of the specimen retrieval pouch is uncoated; and/or wherein at least a portion of an interior surface of the specimen retrieval pouch is coated with a lubricious material.

6. A specimen retrieval pouch according to any preceding claim, wherein the pouch is formed from a hydrophobic material; or wherein the pouch is formed from a porous polymeric material.

7. A specimen retrieval pouch according to claim 6, wherein the exterior surface of the specimen retrieval pouch is modified by chemical solution etching, plasma gas etching, or plasma polymerization deposition.

8. A specimen retrieval pouch according to any preceding claim, wherein the lubricious material is selected from the group consisting of cyanoacrylates, epoxies, polyalkylene oxides, urethanes, siloxanes, and parylenes; preferably wherein the lubricious material includes an anti-microbial additive.

9. A specimen retrieval device, comprising
an applicator; and
a specimen retrieval pouch operably couplable to the applicator, the specimen retrieval pouch including a closed bottom portion and an open upper portion configured to receive a tissue specimen, at least a portion of an exterior surface of the specimen retrieval pouch including a lubricious material.

10. A specimen retrieval device according to claim 9, wherein at least a portion of the exterior surface of the specimen retrieval pouch is coated with the lubricious material.

11. A specimen retrieval device according to claim 9, wherein at least a portion of the pouch is formed from the lubricious material to provide lubricious material on at least a portion of an exterior surface of the specimen retrieval pouch.

12. A specimen retrieval device according to any of claims 9 to 11, wherein the lubricious material is a hydrophilic material.

13. A method comprising:
providing a specimen retrieval pouch including a closed bottom portion and an open upper portion configured to receive a tissue specimen; and
applying a lubricious material to at least a portion of an exterior surface of the open upper portion of the specimen retrieval pouch.

14. A method according to claim 13, wherein the exterior surface of the specimen retrieval pouch is modified by chemical solution etching, plasma gas etching, or plasma polymerization deposition prior to applying the lubricious material.
